# DEMANDE DE BREVET EUROPEEN

(11) **EP 0 534 873 A1**
(43) Date de publication de la demande: **31.03.1993**
(21) Numéro de dépôt: 92420325.0
(22) Date de dépôt: 22.09.1992
(51) Int. Cl.: A61L 27/00, C03C 1/00, C03C 10/00

(54) **Procédé pour la réalisation d'une poudre destinée à la fabrication d'os de synthèse, poudre et produits ainsi réalisés**

(30) Priorité: 26.09.1991 FR 9112083
(71) Demandeur: Pachoud, Christine, F-69110 Sainte Foy Les Lyon (FR); Mai, Christian, F-69006 Lyon (FR)
(72) Inventeur: Pachoud, Christine, F-69110 Sainte Foy Les Lyon (FR); Mai, Christian, F-69006 Lyon (FR)
(74) Mandataire: Laurent, Michel

(57) **Abrégé**

Procédé pour la réalisation d'une poudre destinée à la fabrication d'os de synthèse caractérisé en ce qu'il consiste :
- tout d'abord à faire réagir chimiquement à température ambiante en solution aqueuse deux matrices préparées séparement, respectivement une première matrice à base d'oxyde de titane, de silicium, de zirconium ou de leur association, et une seconde matrice à base de calcium, phosphore, et potassium ;
- puis à faire subir à la poudre ainsi obtenue un premier traitement thermique à une température de 50 °C, jusqu'à obtenir un gel ;
- et enfin, à faire subir à ce gel un second traitement thermique de cristallisation jusqu'à une température d'au moins 800°C.

## Description

L'invention concerne un nouveau procédé pour la réalisation d'une poudre destinée à la fabrication d'os de synthèse. Elle concerne également la poudre ainsi obtenue, de même que les produits obtenus à partir de la poudre en vue de la réalisation d'os de synthèse.

Depuis bien longtemps déjà, la perte osseuse constitue une difficulté dans de nombreux domaines chirurgicaux, notamment orthopédique, maxilo-facial, plastique, bucco-dentaire etc... . A ce jour, différentes alternatives s'offrent au chirurgien. La greffe autologue, autrement dit le prélèvement de greffons sur d'autres éléments osseux du patient, est une solution idéale d'un point de vue compatibilité d'une part, et propriétés mécaniques de la greffe ainsi réalisée d'autre part. Cependant, le capital osseux n'est pas inépuisable, et l'intervention chirurgicale pour collecter un greffon peut créer des traumatismes osseux, particulièrement douleureux pour le patient.

On a alors pensé à effectuer des greffes d'os d'origine humaine, provenant des banques de données d'os. Cette solution pose des problèmes de compatibilité, et s'accompagne également de risques de contamination par virus.

Une autre solution consiste à utiliser des matériaux de synthèse, neutres chimiquement et bio-compatibles. De tels matériaux de substitution doivent présenter d'une part, une résistance mécanique équivalente à celle de l'os, et d'autre part, être susceptibles de développer des propriétés d'adhérence suffisantes au contact de l'os au niveau duquel ils sont mis en place.

Parmi les matériaux de synthèse connus, deux catégories se sont plus particulièrement développées. Pour la première, on peut citer les céramiques à base d'alumine, de zircone ; les bio-verres, les composites carbone-carbone. Les principaux inconvénients de tels matériaux sont tout d'abord la non-ostéoconductricité, réduisant de la sorte les propriétés d'adhérence, et en outre, les différences importantes de leurs propriétés mécaniques, par rapport à celles de l'os humain.

Dans la deuxième catégorie de matériaux de substitution, on peut citer notamment l'hydroxyapatite, les phosphates et les carbonates de calcium, parmi lesquels le corail. De tels matériaux présentent l'avantage par rapport à ceux de la première catégorie d'être ostéoconducteurs. En revanche, leur comportement mécanique s'avère de manière générale insuffisant.

De manière connue, l'os, forme rigide du tissu conjonctif, est formé de cellules et d'une matrice extra-cellulaire. Cette matrice est constituée essentiellement d'une partie minérale contenant du calcium, du phosphore, du magnésium, du potassium, du sodium, du fluor, et d'une partie non minérale contenant du collagène et des protéoglycanes.

Dans les brevets français FR-A-2 585 576 et FR-A-2 564 732, on a suggéré d'associer du collagène contenant du glycosaminoglycane avec de l'hydroxyapatite, ou un facteur ostéoformateur dérivé de l'ostéosarcome du DUNN avec des céramiques. Si certes, les matériaux obtenus présentent une progression dans leurs propriétés, notamment mécaniques par rapport aux matériaux connus à ce jour, les principaux inconvénients reprochés à ceux-ci restent constants, notamment pour les parties minérales et céramiques citées précédemment.

La présente invention vise à pallier ces différents inconvénients. Elle a pour objet de proposer un nouveau bio-matériau de substitution de la matrice osseuse ayant d'une part, une composition chimique minérale voisine de celle de l'os humain, et d'autre part, une structure cristalline et des propriétés mécaniques très proches de celles de l'os. En d'autres termes, le bio-matériau de l'invention présente tous les avantages des matériaux existants, sans leurs inconvénients. L'invention concerne également un procédé pour la réalisation de ce bio-matériau, conférant à celui-ci une structure cristallographique favorisant le phénomène d'ostéogénèse, et par voie de conséquence, l'adhérence de l'os de substitution sur une partie de l'os à substituer.

Ce procédé pour la réalisation d'une poudre destinée à la fabrication d'os de synthèse consiste tout d'abord à faire réagir chimiquement à température ambiante en solution aqueuse deux "matrices" préparées séparément, respectivement une première matrice à base d'oxyde métallique, notamment de titane, de silicium, de zirconium ou de leur association, et une seconde matrice à base de calcium, potassium, phosphore, magnésium, additionnée dans certains cas de fluor et de sodium, puis à faire subir à la poudre ainsi obtenue un premier traitement thermique à une température de 50 °C jusqu'à obtenir un gel, et enfin, à faire subir à ce gel un second traitement thermique de cristallisation à une température d'au moins 800°C, pendant une durée voisine de une heure.

Selon une forme de réalisation de l'invention, on obtient des blocs d'os de synthèse à partir de la poudre ainsi réalisée en soumettant celle-ci à une pression de 2 000 à 4 000 bars, puis en effectuant un frittage de la poudre ainsi compactée à une température comprise entre 900 et 1 100°C.

Selon une forme particulièrement avantageuse de l'invention, on immerge la poudre obtenue dans une solution contenant du collagène de 20 à 50 % en poids à la température ambiante, pendant 24 heures, puis on effectue un filtrage, et enfin une lyophilisation. De la sorte, on obtient une poudre de synthèse favorisant l'ossification entre l'os naturel et l'os de synthèse.

La composition chimique de la première matrice destinée à réaliser la poudre selon l'invention, comporte entre 25 et 45% d'oxyde d'éléments bio-compatibles de silicium, titane, et zirconium, voire d'autres métaux ou de leur association. Si cette concentration est inférieure à 25%, on obtient des propriétés mécaniques plus faibles pour les structures réalisées à partir de cette poudre. En revanche, si cette concentration excède 45%, on observe aucun effet mécanique supplémentaire, et on grève de manière plus important le coût de réalisation de la poudre.

La deuxième matrice, préparée indépendamment de la première matrice, est une solution aqueuse comportant en moyenne de 30 à 40 % de calcium, 10 à 20 % de phosphore, 2 à 6 % de magnésium, 0 à 1 % de potassium ainsi que le sodium, et 0 à 2 % de fluor. Le pourcentage de la deuxième matrice est complémentaire de celui de la première matrice, puisque dans tous les cas, on ajuste la composition de cette seconde matrice à 100 %.

L'obtention de la poudre selon l'invention s'effectue par réaction chimique en ajoutant la seconde matrice dans la première matrice sous agitation mécanique à la température ambiante. Le mélange est ensuite chauffé lentement à 50 °C et maintenu à cette température jusqu' à la formation d'un gel. Celui-ci est alors soumis à un traitement thermique pendant environ une demi-heure à 300 °C, puis cristallisé par traitement thermique à 800°C pendant environ deux heures.

La manière dont l'invention peut être réalisée et les avantages qui en découlent, ressortiront mieux des exemples de réalisation qui suivent donnés à titre indicatif et non limitatif à l'appui des figures annexées.

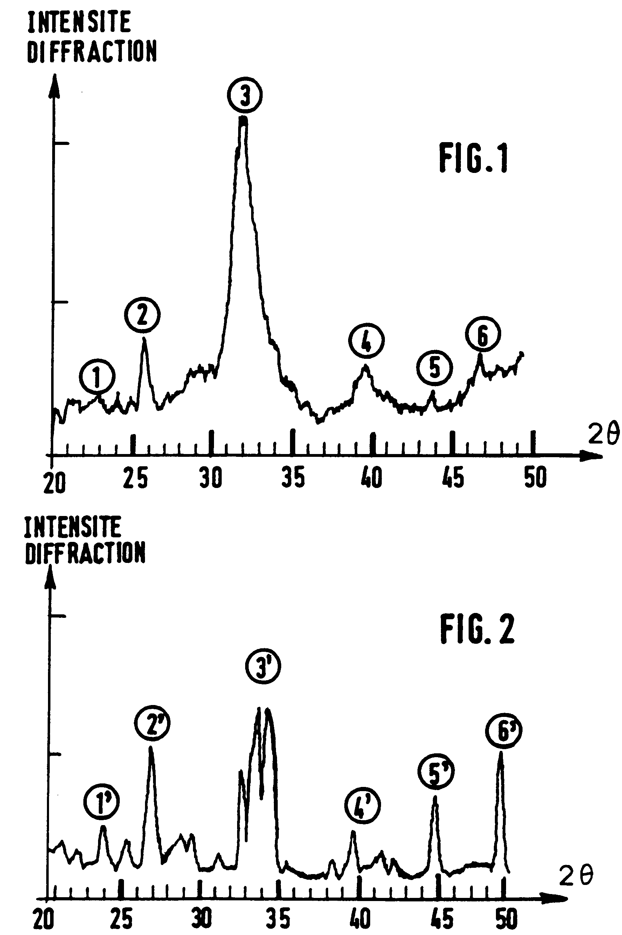

Les figures 1, 2, 3 et 4 sont respectivement des diagrammes de diffraction aux rayons X d'un os cortical frais, de la poudre obtenue conformément à l'invention, du corail et de l'hydroxyapatite.

### Exemple 1

On procède tout d'abord à la synthèse de la première matrice conformément à la méthode de l'invention. On mélange une solution d'alcoxyde de silicium avec une solution d'eau alcoolisée, selon la proportion d'une mole d'alcoxyde pour quatre moles d'eau, en présence d'acide, de manière à modifier la solution en l'amenant à un pH inférieur à 7, ou en présence de base pour obtenir une solution de pH supérieur à 7. Dans tous les cas, le pH doit être différent de la neutralité, afin que les réactions chimiques ultérieures puissent avoir lieu.

Ce mélange est ensuite agité pendant 15 minutes à température ambiante pour empêcher la prise en masse trop rapide. On obtient de la sorte une solution transparente.

Parallèlement, on procède à la synthèse de la deuxième matrice. Celle-ci s'effectue à partir d'une solution aqueuse contenant en pourcentage molaire :
- 37 % de calcium sous forme de nitrate de calcium CaNo₃ ou de chlorure de calcium CaCl₂,
- 16 % de phosphore sous forme d'acide phosphorique H₂PO₄,
- 0,5 % de magnésium sous forme de chlorure de magnésium MgCl₂,
- 0,1 % de potassium sous forme de carbonate de potassium K₂CO₃,
- et enfin 0,05% de sodium sous forme de chlorure de sodium NaCl.

On procède alors au mélange de la deuxième matrice dans la première matrice sous agitation mécanique, et on laisse la réaction chimique se poursuivre à la température de 50°C, jusqu'à la formation d'un gel humide. Ce gel subit ensuite un cycle de traitement thermique à 300 °C, puis une cristallisation jusqu'à une température de 800°C, à une vitesse de montée en température de l'ordre de 10°C par minute. On obtient de la sorte une poudre qui est ensuite lavée dans l'eau distillée Jusqu'à ce qu'à obtenir la neutralité de la solution avec un pH égal à 7. La poudre est ensuite séchée par chauffage à une température voisine de 300°C.

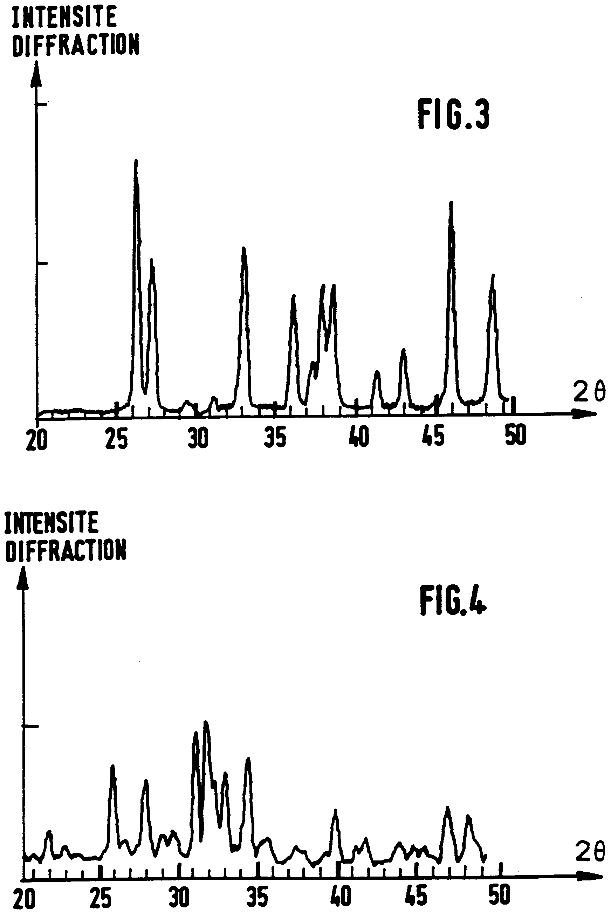

On observe par comparaison des figures 1 et 2 une remarquable similitude des deux diagrammes de diffraction des rayons X, indiquant de la sorte une grande ressemblance quant à la structure cristalline de la poudre obtenue par rapport à celle d'un os cortical humain. On a référencé par (1, 2, 3, 4, 5 et 6) les pics caractéristiques de l'os cortical frais sur la figure 1, et par ( 1′, 2′, 3′, 4′, 5′ et 6′) ceux représentatifs de la poudre de synthèse selon l'invention. A titre de comparaison, on a représenté sur les figures 3 et 4 respectivement les diagrammes de diffraction d'un corail et d'un phosphate de calcium : l'hydroxyapatite, couramment utilisés comme déjà dit en chirurgie orthopédique. On constate que les structures cristallines de ces matériaux sont très différentes de celles de l'os humain, expliquant en partie leurs différences de comportement notamment mécaniques.

La poudre ainsi obtenue s'avère particulièrement adaptée notamment dans le cadre des comblements osseux.

Lorsqu'il s'agit de pratiquer des ostéotomies, il importe de compacter cette poudre sous forme de bloc. Ces blocs de synthèse selon l'invention sont obtenus par compactage entre 2 000 et 4 000 bars de la poudre ainsi obtenue, puis par frittage des blocs ainsi obtenus, entre 900 et 1100 °C. La porosité de ces blocs est fonction de la compaction de la poudre et de la température de frittage. Les blocs ainsi obtenus ont une dureté Vickers de l'ordre de Hᵥ = 450 et un module d'Young voisin de E = 30 GPa. A titre comparatif, la dureté Vickers de l'os cortical est Hᵥ = 430, et son module d'Young est de E = 20 GPa.

La poudre et les blocs d'os de synthèse ainsi obtenus par cet exemple, représentent la partie minérale de l'os humain. Ils peuvent être avantageusement utilisés tel quel dans diverses applications chirurgicales.

### Exemple 2

On procède tout d'abord à la synthèse de la première matrice, de manière identique à celle de l'exemple 1.

La seconde matrice est obtenue à partir d'une solution aqueuse contenant en pourcentage molaire :
- 37% de calcium (nitrate de calcium CaNO₃ ou chlorure de calcium CaCl₂),
- 16% de phosphore : acide phosphorique H₂PO₄,
- 0,5 % de magnésium (chlorure de magnésium MgCl₂),
- 0,1 % de potassium (carbonate de potassium K₂CO₃),
- et 2% de fluor (fluorure de calcium CaF₂).

L'obtention de la poudre d'os de synthèse se fait dans les mêmes conditions que dans l'exemple 1, par mélange des deux matrices ainsi obtenues en solution aqueuse puis lavage à l'eau distillée, séchage et traitement thermique.

De même, les blocs d'os de synthèse sont réalisés dans les mêmes conditions que pour l'exemple 1.

La poudre ainsi obtenue est mieux adaptée pour les applications bucco-dentaires, notamment de par la présence des ions fluor présents dans la composition.

### Exemple 3

La première matrice est réalisée avec un mélange d'une solution d'alcoxyde de titane avec une solution d'eau alcoolisée dans les proportions d'une mole d'alcoxyde pour 4 moles d'eau en présence d'acide chlorhydrique de manière à obtenir une solution avec un pH inférieur à 7. Ce mélange est ensuite agité pendant quinze minutes à une température inférieure à la température ambiante.

La préparation de la seconde matrice s'effectue dans les mêmes conditions que celles indiquées dans les exemples 1 et 2.

L'obtention de la poudre d'os de synthèse s'effectue également dans les mêmes conditions que celles précisées dans les exemples 1 et 2.

On obtient les mêmes caractéristiques physico-chimiques et mécaniques que celles décrites dans l'exemple 1 ou 2. De la même manière, on obtient des blocs d'os de synthèse selon les mêmes conditions que celles indiquées dans l'exemple 1.

L'introduction d'une matrice à base de titane s'avère particulièrement avantageux dans le cadre d'application de la poudre ou des blocs réalisés à partir de celle-ci sur des supports en titane, tels que par exemple des prothèses de hanche, genou, etc.. En effet, la présence de titane, connu en outre pour sa bio-compatibilité, favorise l'accrochage de la poudre sur de tels supports.

### Exemple 4

La préparation de la première matrice, à base d'une solution d'alcoxyde de silicium avec une solution d'eau alcoolisée s'effectue cette fois dans les proportions de une mole d'alcoxyde pour 12 moles d'eau en présence d'acide chlorhydrique, pour amener la solution à un pH inférieur à 7.

Ce mélange est ensuite agité pendant quinze minutes à une température de 20°C. On ajoute alors à cette solution un alcoxyde de titane selon la proportion de deux moles d'alcoxyde de titane pour une mole d'alcoxyde de silicium. Le tout est agité pendant 15 mn à 20°C.

La préparation de la seconde matrice s'effectue dans les mêmes conditions que celles indiquées dans l'exemple 1.

L'obtention de la poudre et des blocs s'effectue dans les mêmes conditions que celles indiquées dans l'exemple 1.

Cette poudre est un composite, dont les propriétés sont intermédiaires entre les produits obtenus selon les exemples 1 et 3.

### Exemple 5

On obtient une poudre selon les mêmes conditions que celles indiquées dans les exemples 1 à 4. La poudre ainsi obtenue est ensuite remise en solution dans un solvant contenant entre 20 et 50% en volume de collagène. L'ensemble ainsi obtenu est agité à température ambiante pendant 30 minutes environ. On laisse la poudre s'imprégner par le collagène pendant 24 heures, puis on sépare la poudre par filtration. La poudre ainsi filtrée est alors lavée et soumise à lyophilisation.

La synthèse de la poudre au collagène s'effectue dans les mêmes conditions que celles indiquées dans les exemples ci-dessus décrits. Elle peut être ensuite compactée sous une pression comprise entre 2 000 et 4 000 bars, mais à la température ambiante afin de préserver le collagène.

La poudre au collagène ainsi obtenue favorise l'épithélialisation. En outre, le compactage de la poudre, et la tenue mécanique des blocs non frittés est meilleure.

Les premières expérimentations effectuées avec les poudres et blocs obtenus selon l'invention montrent aucun phénomène d'intolérance à court, moyen et long terme (un an). En effet, il n'a jamais été observé :
- de réactions inflammatoires aigües ou chroniques;
- de réactions infectieuses à polynucléaires ;
- d'encapsulations fibreuses.

En outre, eu égard aux propriétés ostéoconductrices du matériau ainsi réalisé d'une part, et à la compatibilité de ce bio-matériau avec les tissus qu'il est sensé compléter d'autre part, on observe une très bonne ossification, notamment à l'interface entre le bio-matériau et l'os naturel sur lequel il est greffé. En d'autres termes, on observe un agrafage osseux avec aspect radiologique homogène et dense. Le bio-matériau ainsi obtenu, pour lequel l'invention n'est nullement limitée aux formes de réalisation décrites et est donc susceptible de diverses variantes sans sortir de son cadre, s'avère dont particulièrement avantageux, notamment dans le cadre de la chirurgie orthopédique, maxilo-faciale, bucco-dentaire, esthétique.

## Revendications

**1/** Procédé pour la réalisation d'une poudre destinée à la fabrication d'os de synthèse caractérisé en ce qu'il consiste :
- tout d'abord à faire réagir chimiquement à température ambiante en solution aqueuse deux matrices préparées séparement, respectivement une première matrice à base d'oxyde de titane, de silicium, de zirconium ou de leur association, et une seconde matrice à base de calcium, phosphore, et potassium ;
- puis à faire subir à la poudre ainsi obtenue un premier traitement thermique à une température de 50 °C, jusqu'à obtenir un gel ;
- et enfin, à faire subir à ce gel un second traitement thermique de cristallisation jusqu'à une température d'au moins 800°C.

**2/** Procédé selon la revendication 1, caractérisé en ce que le second traitement thermique que subit le gel est précédé d'un pré-traitement à 300 °C pendant une demi-heure.

**3/** Procédé pour la réalisation de blocs d'os de synthèse à partir de la poudre obtenue selon le procédé de l'une des revendications 1 et 2, caractérisé en ce qu'il consiste tout d'abord à compacter la poudre ainsi obtenue sous une pression comprise entre 2 000 et 4 000 bars, puis à fritter les blocs ainsi obtenus sous une température comprise entre 900 et 1 100 °C.

**4/** Procédé pour la réalisation d'une poudre pour la fabrication d'os de synthèse selon l'une des revendications 1 et 2, caractérisé en ce que la poudre ainsi obtenue est remise en solution avec du collagène selon une concentration de 20 à 50% en volume de la solution à température ambiante pendant vingt-quatre (24) heures, puis est filtrée et enfin lyophilisée.

**5/** Procédé pour la fabrication de blocs d'os de synthèse à partir de la poudre obtenue selon la revendication 4, caractérisé en ce qu'il consiste à compacter la poudre ainsi obtenue sous une pression comprise entre 2 000 et 4 000 bars à température ambiante.

**6/** Poudre et blocs d'os de synthèse obtenus selon le procédé des revendications 1 à 5, caractérisés en ce que leur concentration molaire en oxyde de titane, de silicium, de zirconium ou de leur association est comprise entre 25 et 45%, et en ce que la concentration molaire en calcium, phosphore, potassium est comprise entre 55 et 75%, la somme de ces deux pourcentages étant égale à 100%, la structure cristalline de la poudre et des blocs étant très voisine de celle de l'os naturel.

**7/** Poudre et blocs d'os de synthèse selon la revendication 6, caractérisés en ce que la concentration molaire du calcium est comprise entre 30 et 40%, celle du phosphore entre 10 et 20%, celle du potassium entre 0 et 1%, la poudre et les blocs comportant également du magnésium à raison de 2 et 6 % en mole, du fluor à raison de 0 et 2% en mole, et du sodium à raison de 0 à 1% en mole.

**8/** Poudre et blocs de synthèse selon l'une des revendications 6 et 7, caractérisés en ce qu'ils comportent en outre du collagène à raison de 20 à 50 % en volume.
